# EUROPÄISCHE PATENTSCHRIFT

(11) **EP 2 007 276 B1**
(45) Veröffentlichungstag und Bekanntmachung des Hinweises auf die Patenterteilung: **16.11.2011**
(21) Anmeldenummer: 07722098.6
(22) Anmeldetag: 23.03.2007
(51) Int. Cl.: A61B 5/04, A61B 5/0476, A61B 5/0482

(54) **VORRICHTUNG ZUR MESSUNG BIOMEDIZINISCHER DATEN EINES PROBANDEN UND VERFAHREN ZUR STIMULATION DES PROBANDEN MIT IN ECHTZEIT VERARBEITETEN DATEN**
DEVICE FOR MEASURING BIOMEDICAL DATA FROM A TESTEE AND METHOD FOR STIMULATING THE TESTEE WITH DATA PROCESSED IN REAL-TIME
DISPOSITIF DE MESURE DE DONNEES BIOMEDICALES PROVENANT D'UN SUJET A EXAMINER ET PROCEDE PERMETTANT LA STIMULATION D'UN SUJET A EXAMINER AU MOYEN DE DONNEES TRAITEES EN TEMPS REEL

(30) Priorität: 15.04.2006 DE 102006017716
(43) Veröffentlichungstag der Anmeldung: 31.12.2008
(73) Patentinhaber: Forschungszentrum Jülich GmbH, 52425 Jülich (DE)
(72) Erfinder: RONGEN, Heinz, 52353 Düren (DE); ZIEMONS, Karl, 52076 Aachen (DE); SCHIEK, Michael, 52066 Aachen (DE); TASS, Alexander, Prof. Dr. Dr., 81541 München (DE)
(86) Internationale Anmeldenummer: PCT/DE2007/000539
(87) Internationale Veröffentlichungsnummer: WO 2007/118443

(56) Entgegenhaltungen:
- DE-A1- 4 030 782
- US-A- 5 099 856
- US-A- 5 331 969
- US-A- 5 445 162
- US-A- 5 581 387
- POZZO M ET AL: "SIXTY-FOUR CHANNEL WEARABLE ACQUISITION SYSTEM FOR LONG-TERM SURFACE ELECTROMYOGRAM RECORDING WITH ELECTRODE ARRAYS" MEDICAL AND BIOLOGICAL ENGINEERING AND COMPUTING, SPRINGER, HEILDELBERG, DE, Bd. 42, Nr. 4, Juli 2004 (2004-07), Seiten 455-466, XP001221029 ISSN: 0140-0118

## Beschreibung

Die Erfindung betrifft eine Vorrichtung zur Messung biomedizinischer Daten eines Probanden und ein Verfahren zur Stimulation des Probanden mit in Echtzeit verarbeiteten Daten.

Geräte, die als Mehrkanalschreiber ein elektromagnetisches Korrelat der neuronalen Aktivität eines Probanden in Abhängigkeit von der Zeit aufnehmen, sind seit vielen Jahren Stand der Technik. Beispielhaft seien Elektroencephalographen (EEG) oder Magnetoencephalographen (MEG) genannt.

Diese Geräte weisen eine Vielzahl an Messkanälen auf. Die Geräte eignen sich nicht nur zur Signalerfassung sondern auch zur Speicherung und Auswertung von Daten. Die Auswertung von Daten erfolgt im offline-Modus des Gerätes, das heißt zu einem Zeitpunkt, an dem keine Daten am Probanden erhoben werden. Hierzu werden die durch das Gerät erhobenen Daten an eine Workstation weitergeleitet, abgespeichert und gegebenenfalls auch weiterverarbeitet.

Die Schriften US 5,099,856 A, US 5,331,969 A und US 5,445,162 A beschreiben Vorrichtungen zur Messung biomedizinischer Daten eines Probanden. US 5,445,162 offenbart die Merkmale des Oberbegriffs von Auspruch 1 .

Aus Rongen et al. (H. Rongen, V. Hadamscheck, M. Schiek (2005). Real-Time Data Acquisiton and Online Signal Processing for Magnetoencephalography. In: Conference proceedings zu IEEE-NPSS Real Time Conference 2005, June 4-10, Stockholm, Sweden, P5-7.) ist bekannt, mittels eines 148-Kanal Magnetoencephalographen Datensätze zu erheben und für eine 3D-Rekonstruktion des Gehirns zu speichern, zu verarbeiten und zu visualisieren. Nachteilig ist damit eine Verarbeitung und Auswertung der Daten, schritthaltend zur laufenden Messung der Daten am Probanden in Echtzeit nicht möglich.

Aufgabe der Erfindung ist es, eine Vorrichtung zur Messung biomedizinischer Daten eines Probanden bereit zu stellen, welche eine Aufzeichnung und Verarbeitung der Daten in Echtzeit gewährleistet. Eine weitere Aufgabe der Erfindung ist es, ein Verfahren zur Nutzung derartig verarbeiteter Daten anzugeben.

Die Aufgabe wird durch eine Vorrichtung gemäß Hauptan-Anspruch 1 und ein Verfahren gemäß Anspruch 10 gelöst. Vorteilhafte Ausgestaltungen ergeben sich aus den jeweils darauf rückbezogenen Patentansprüchen.

Die Vorrichtung umfasst ein Messsystem zur Erhebung der Daten sowie eine erste Hardware-Komponente zur Aufzeichnung der Daten. Zwischen dem Messsystem und der ersten Hardware-Komponente ist eine Verbindungsleitung zur Übertragung der Daten angeordnet. In der Verbindungsleitung zwischen der ersten Hardware-Komponente zur Aufzeichnung der Daten und dem Messsystem zur Erhebung der Daten ist erfindungsgemäß ein Mittel zur galvanischen Auftrennung der Daten angeordnet.

Durch das Mittel zur galvanischen Auftrennung wird vorteilhaft eine elektrische Entkopplung des Probanden nebst Messsystem zur Erhebung der Daten von den übrigen, nachgelagerten Hardware-Komponenten zur Aufzeichnung und Verarbeitung der Daten bewirkt. Dies ist nach dem Medizinproduktegesetz und in Übereinstimmung mit den grundlegenden Anforderungen der MDD 93/42 EWG gefordert.

Dadurch wird eine Schädigung des Probanden im Falle einer elektrischen Störung dieser nachgelagerten Hardware-Komponenten vermieden. Auch das Messsystem zur Erhebung der Daten selbst ist selbstverständlich vor Störungen geschützt.

Das Messsystem zur Erhebung der Daten umfasst im Falle eines Elektroencephalographen oder eines Magnetoencephalographen die Elektroden, die Messkanäle und andere gerätespezifische Komponenten, wie Sensoren und so weiter. Diese können je nach Anbieter eines Gerätes variieren.

Als Proband muss nicht notwendigerweise ein Mensch vorgesehen sein. Vielmehr kann auch jedes Tier und insbesondere jedes Säugetier als Proband dienen.
Es ist selbstverständlich auch möglich, biologisch aktive Hirn- bzw. Gewebeschnitte eines Probanden auf die vorliegend offenbarte Weise zu untersuchen bzw. zu stimulieren.

Das Mittel zur Auftrennung der Daten gewährleistet vorteilhaft die störungsfreie Auftrennung und Kopie der Daten eines Datensatzes in mindestens zwei identische Datensätze.
Durch eine störungsfreie Auftrennung der Daten wird vorteilhaft bewirkt, dass der Vorgang der Auftrennung auf das Messsystem.zur Erhebung der Daten und somit auf die erhobenen Daten selbst keinen Einfluss hat.

Durch die störungsfreie Auftrennung der Daten selbst bleiben somit sowohl die Daten als auch der Proband vollkommen unbeeinflusst.

Es ist möglich, eine erfindungsgemäße Vorrichtung mit einem erfindungsgemäßen Mittel derartig auszugestalten, dass dieses die durch das Messsystem erhobenen Daten in mehr als zwei identische Datensätze auftrennt. Die durch das Messsystem erhobenen Daten sind jedenfalls durch die Auftrennung der Daten unverändert.

Die Verbindungsleitung zwischen Messsystem und erster Hardware-Komponente wird besonders vorteilhaft durch einen Lichtwellenleiter gebildet, in dem ein Lichtwellenleiterkoppler als erfindungsgemäßes Mittel angeordnet ist.

Es ist möglich, an Stelle eines Lichtwellenleiters eine Kupferleitung zwischen Messsystem und erster Hardware-Komponente als Verbindungsleitung anzuordnen, in der ein galvanischer Trennverstärker als erfindungsgemäßes Mittel angeordnet ist.

Die erste Hardware-Komponente wird z. B. durch eine Workstation gebildet. Diese steuert gemäß Stand der Technik regelmäßig den Messablauf eines Messsystems zur Erhebung der Daten. Zu letztgenanntem Zweck ist eine weitere Verbindungsleitung zwischen der ersten Hardware-Komponente und dem Messsystem zur Erhebung der Daten vorgesehen.

Das Messsystem zur Erhebung der Daten und die erste Hardware-Komponente nebst einer Verbindungsleitung zwischen dem Messsystem und der ersten Hardware-Komponente stellen in Bezug auf Elektroencephalographen (EEG) oder Magnetoencephalographen (MEG) den Stand der Technik dar.
Das Mittel zur galvanischen Auftrennung der Daten in der Verbindungsleitung erweitert derartige Gerätschaften und bewirkt, dass die Daten nicht nur in der ersten Hardware-Komponente gespeichert, sondern auch in einer zweiten Hardware-Komponente in Echtzeit weiterverarbeitet werden können.

Vorteilhaft können somit die durch das Messsystem erhobenen Daten, neben der Aufzeichnung und langfristigen Speicherung in der ersten Hardware-Komponente, auch noch mindestens einer weiteren zweiten Hardware-Komponente zur Verarbeitung der Daten in Echtzeit zugeführt werden.

Das erfindungsgemäße Mittel zur Auftrennung der Daten ist vorteilhaft als ein passives Mittel ausgelegt. Dadurch wird bewirkt, dass die zweite Hardware-Komponente zur Verarbeitung und Speicherung der Daten nur am bestehenden Datenstrom zur ersten Hardware-Komponente "mithorcht" ohne aktiv in den Datenstrom einzugreifen.

Die zweite Hardware-Komponente verhält sich passiv zum Datenstrom.

Das Mittel zur Auftrennung der Daten ist dann derartig in der vom Messsystem zur Erhebung der Daten am Probanden abgehenden Verbindungsleitung angeordnet, dass es den Datenstrom vom Messsystem zur Erhebung der Daten auf zwei Verbindungsleitungen auftrennt. Eine vom erfindungsgemäßen Mittel abgehende Leitung leitet den Datenstrom zu der ersten Hardware-Komponente zur Aufzeichnung der Daten weiter. Mindestens ein weiterer Datenstrom wird zur zweiten Hardware-Komponente weitergeleitet.
Auf diese Weise wird ein vollständiger, identischer Datensatz auch mindestens der zweiten Hardware-Komponente zugeführt.

Die zweite Hardware-Komponente verarbeitet die Daten in Echtzeit und speichert die einkommenden als auch die verarbeiteten Daten. Die zweite Hardware-Komponente führt auch die Steuerung des Messsystems zur Erhebung der Daten durch.

Die Echtzeit-Datenverarbeitung in der zweiten Hardware-Komponente ermöglicht besonders vorteilhaft ein Verfahren zur Stimulation des Probanden mit den verarbeiteten Daten mittels Rückführung bzw. Weiterleitung der verarbeiteten Daten zum Probanden in Echtzeit..

In einer Ausgestaltung der Erfindung umfasst die zweite Hardware-Komponente zur Verarbeitung der Daten eine PCI-Basisplatine, auf der die Komponenten für die Echtzeit-datenverarbeitung und Speicherung von einkommenden und verarbeiteten Daten angeordnet sind. Die PCI-Basisplatine erweitert hierzu einen PC.

Die PCI-Basisplatine umfasst zu diesem Zweck vorteilhaft die Bausteine bzw. Komponenten einer Datenerfassungs- und Verarbeitungseinheit, und insbesondere auch diejenigen für die Erzeugung von Rückkopplungssignalen an den Probanden. Die PCI-Basisplatine weist hierzu eine leistungsfähige Echtzeitdatenerfassungs-und Verarbeitungseinheit auf. Darüber hinaus weist die PCI-Basisplatine selbstverständlich weitere Komponenten zur Speicherung und gegebenenfalls Steuerung des Messsystems zur Erhebung der Daten auf.

Die PCI-Basisplatine der zweiten Hardware-Komponente umfasst besonders vorteilhaft einen digitalen Signalprozessor (DSP), z. B. einen Texas Instruments TMS320c6713 zur Verarbeitung der Daten in Echtzeit.

Die PCI-Basisplatine der zweiten Hardware-Komponente umfasst in einer weiteren Ausgestaltung der Erfindung einen field programmable Gate Array (FPGA), z. B. einen Virtex XCV300.

Die zweite Hardware-Komponente muss aber nicht notwendigerweise einen field programmable Gate Array (FPGA) umfassen.
Die zweite Hardware-Komponente muss nur leistungsfähig genug sein, um die einkommenden Daten zu erfassen und nötige Berechnungen für die Erzeugung von Rückkopplungssignalen, Schritt haltend zur Erhebung der Daten durch das Messsystem, durchzuführen. Die zweite Hardware-Komponente zur Verarbeitung der Daten umfasst auch die notwendigen Ausgänge zur Weiterleitung der vom Messsystem erhobenen, verarbeiteten Daten an den Probanden.

Mittels geeigneter Algorithmen, welche im digitalen Signalprozessor (DSP) abgelegt sein können, werden aus den verarbeiteten Daten Rückkopplungssignale zur Stimulation des Probanden, Schritt haltend mit der Messung der Daten durch das Messsystem, generiert und über geeignete Verbindungsleitungen an den Probanden zurückgeführt.

Die PCI-Basisplatine umfasst vorteilhaft eine PCI-Bus-Schnittstelle sowie die nötigen Ein- und Ausgabekömponenten.

Dies sind im Falle von Lichtwellenleitern neben einer Lichtwellenleiter-Schnittstelle auch analoge und digitale Ein- und Ausgänge.
Damit bietet diese Datenerfassungs- und Verarbeitungseinheit alle notwendigen Schnittstellen zur Anbindung an das Messsystem zur Erhebung der Daten, wie z. B. an ein Magnetoencephalograph oder an ein Elektroencephalograph.

Mittels der zweiten Hardware-Komponente werden die Daten durch Algorithmen verarbeitet, welche auf der PCI-Basisplatine im FPGA und im DSP gespeichert sein können.

Über eine dritte Hardware-Komponente werden die auf den Messdaten basierenden, in der zweiten Hardware-Komponente verarbeiteten Daten, als Rückkopplungssignale an den Probanden zwecks Stimulation weitergeleitet.
Dies geschieht besonders vorteilhaft Schritt haltend mit der Datenerhebung des Messsystems und der Verarbeitung der Daten in der zweiten Hardware-Komponente.

Die Weiterleitung der verarbeiteten Daten an den Probanden ist dabei um eine feste oder um eine determiniert variable, bekannte Verzögerung gegenüber der Signalerfassung zeitlich verzögert, so dass ein Proband, basierend auf aktuell erhobenen und verarbeiteten Messsignalen, stimuliert werden kann.

Die dritte Hardware-Komponente umfasst hierzu eine optische Stimulationsbrille.
Die dritte Hardware-Komponente zur Stimulation umfasst dann z. B. eine lineare Ansteuerung einer Lichtquelle zur visuellen Stimulation.

Es kann aber auch eine akustische Stimulation des Probanden mit den von ihm erhobenen und verarbeiteten Daten vorgesehen sein.
Hierzu umfasst die dritte Hardware-Komponente vorteilhaft eine Tonquelle, welche in Lautstärke und/oder der Frequenz mittels analoger Ausgangssignale an der zweiten Hardware-Komponente angesteuert werden kann. Das akustische Signal wird dem Probanden über einen Lautsprecher oder Kopfhörer zugeführt, so dass dieser wiederum stimuliert wird.

Mittels der erfindungsgemäßen Vorrichtung wird vorteilhaft eine störungsfreie Erweiterung bereits vorhandener Messsysteme und Geräte, wie Magnetoencephalographen oder Elektroencephalographen, bereitgestellt, wodurch diese zur elektrischen und magnetischen Stimulation des Probanden erweitert werden.
Die Geräte werden erfindungsgemäß um Komponenten für echtzeitfähige Rückkopplungen erweitert. Hierzu kann jedes handelsübliche Messsystem um ein erfindungsgemäßes Mittel zur galvanischen Auftrennung erhobener Daten und zur Verarbeitung der Daten erweitert werden.

Ein erfindungsgemäßes Verfahren zur Stimulation eines Probanden weist die nachfolgenden Schritte auf:
a) Daten eines Probanden werden durch ein Messsystem erhöben,
b) die erhobenen Daten werden galvanisch getrennt und eine Kopie des Datensatzes wird einer Echtzeitdatenverarbeitungseinheit zugeführt,
c) die Daten werden in Echtzeit verarbeitet,
d) die verarbeiteten Daten werden in Echtzeit zum Zweck des Stimulation an den Probanden weitergeleitet, wobei zur Stimulation eine Stimulationsbrille und/oder eine Tonquelle eingesetzt werden.
Die verarbeiteten Daten in d) werden in Form analoger Signale an den Probanden weitergeleitet.

Die Schritte a) bis d) werden vielfach, z. B. etwa tausendmal je Sekunde wiederholt.

Mittels des erfindungsgemäßen Verfahrens werden die Daten linear oder nichtlinear transformiert und zeitlich verzögert an den Probanden zurückgeführt. Die nicht-lineare Transformation kann dabei Daten verschiedener Zeiten miteinander verknüpfen.

Zur Durchführung des Verfahrens ist eine erfindungsgemäße Vorrichtung besonders geeignet. Es handelt sich dann im Sinne der Erfindung bei diesen Gerätschaften regelmäßig um MEG- oder EEG-online Systeme, das heißt um Systeme in denen die Daten erhoben, galvanisch getrennt, verarbeitet und an den Probanden weitergeleitet werden.

Durch die Verwendung der leistungsfähigen Echtzeitdatenerfassungs- und Signalverarbeitungseinheit in Kombination bzw. Erweiterung eines handelsüblichen PC wird die benötigte Rechenleistung zur Verfügung gestellt, um in Echtzeit die Rückkopplungssignale, basierend auf den erhobenen Daten, zu berechnen und diese wieder zur Stimulation des Probanden auszugeben.

Im Weiteren wird die Erfindung an Hand zweier Ausführungsbeispiele und der beigefügten Figuren näher beschrieben.

Es zeigen:
Fig. 1: Schematische Darstellung eines erfindungsgemäßen MEG-online Systems.
Fig. 2: Lichtwellenleiterkoppler als erfindungsgemäßes Mittel zur galvanischen Auftrennung der Daten und dessen Anschlüsse.
Fig. 3: Schaltbild der PCI-Basisplatine der zweiten Hardware-Komponente mit Bausteinen.
Fig. 4: Schaltbild des Ein- und Ausgabemoduls der PCI-Basisplatine.

Identische Bezugszeichen in den Figuren bezeichnen identische Bauteile bzw. Komponenten.

Erstes Ausführungsbeispiel:

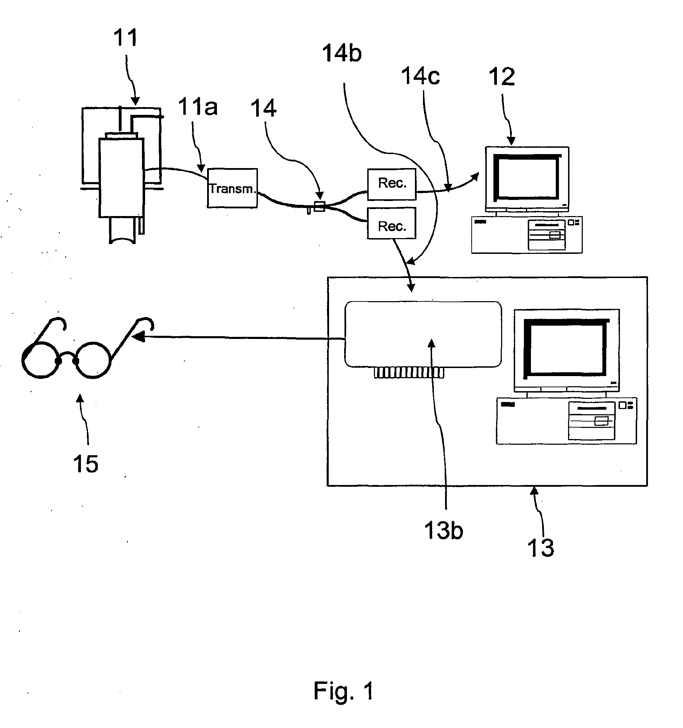

In Fig. 1 ist links oben ein Mehrkanal-MEG 11 mit insgesamt 148 Einzelkanälen als ein Messsystem zur Erhebung der Daten dargestellt. Das Mehrkanal-MEG 11 ist handelsüblich über einen Lichtwellenleiter 11a mit einer Workstation 12 als erste Hardware-Komponente unmittelbar verbunden und z. B. von der Fa. 4D-Neuroimaging, Modell Magnis 2500 WH, erhältlich. Die Workstation 12 hat die Funktion, die einkommenden Daten abzuspeichern. Im offline-Modus werden gemäß Stand der Technik zu einem späteren Zeitpunkt, nach der Datenerhebung durch das Messsystem, die Daten ausgewertet.

Die Workstation 12 hat darüber hinaus auch die Funktion das Messsystem 11 in Bezug auf dessen Messablauf zu steuern. Hierzu ist eine weitere Verbindungsleitung zwischen Workstation 12 und Messsystem 11 angeordnet (nicht dargestellt).

Dieses handelsübliche Messsystem 11 wird erfindungsgemäß über einen Lichtwellenleiterkoppler 14 mit einer zweiten Hardware-Komponente 13 mit Echtzeitdatenerfassungs- und Signalverarbeitungseinheit 13b verbunden.

Für diese störungsfreie Ankopplung an das bestehende MEG-Messsystem 11 mit Workstation 12 wird der Lichtwellenleiterkoppler 14 in den vom Messsystem 11 abgehenden Lichtwellenleiter 11a angeordnet.

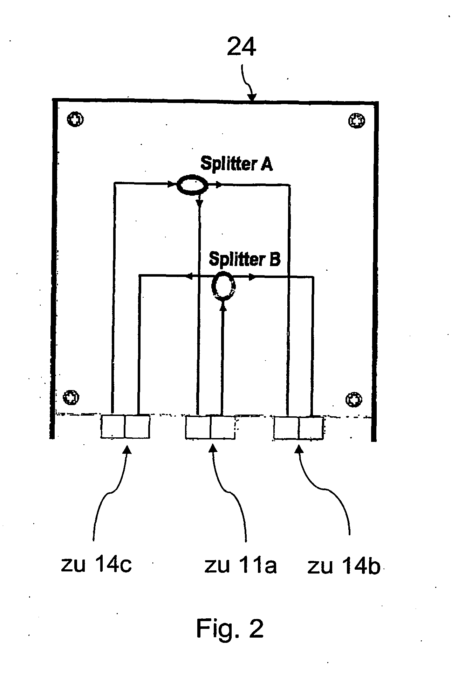

Die Signalführung im Lichtwellenleiterkoppler 14, 24 und das Auftrennen eines eingehenden optischen Signals auf zwei Ausgänge ist in Figur 2 dargestellt.

Der verwendete Lichtwellenleiterkoppler 14, 24 ist ein Multimode-Koppler 1 x 2 - G62,5 / 125 der Fa. TEDIS.

Der Lichtwellenleiterkoppler 14, 24 stellt eine optische, passive Kopplung der Lichtwellenleiterfasern der Leitung 11a mit denen der Leitungen 14b und 14c dar. Er weist Anschlüsse für den Lichtwellenleiter 11a vom Messsystem 11, sowie zwei Anschlüsse für die Lichtwellenleiter 14c und 14b zur ersten Hardware-Komponente 12 und zur zweiten Hardware-Komponente 13 auf. Damit kann der Lichtwellenleiterkoppler 14, 24 durch Einstecken der entsprechenden Kabel mit Steckern leicht in das bestehende Magnetoencephalograph eingefügt werden.

Der Lichtwellenleiterkoppler 14, 24 bewirkt die galvanische Auftrennung des Datenstroms ausgehend von der MEG-Sensorelektronik (nicht dargestellt) im Messsystem 11.

Der Lichtwellenleiterkoppler 14, 24 teilt den Datenstrom vom Messsystem 11 über die Leitung 11a in zwei identische und vollständige Datensätze auf die beiden Leitungen 14c und 14b auf. Der Datenstrom wird somit in zwei unveränderte, zueinander identische Datensätze kopiert und in Form optischer Signale auf die beiden Ausgänge am Lichtwellenleiterkoppler 14, 24 geleitet.

Beide Hardware-Komponenten 12 und 13 erhalten störungsfrei einen kompletten Satz der durch das MEG-Messsystem 11 vom Probanden erhobenen Daten. Die Leitung 14c führt zur Workstation 12, in der die Daten kontinuierlich aufzeichnet werden. Die Leitung 14b führt zu der zweiten Hardware-Komponente 13 (rechts unten in Fig. 1), in der die Daten in Echtzeit verarbeitet und zur Erzeugung von Stimulationssignalen für den Probanden an diesen weitergeleitet werden.

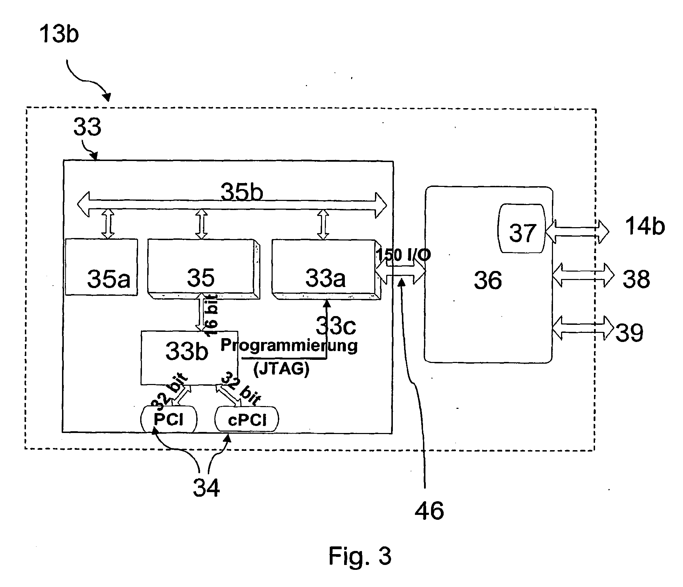

Fig. 3 zeigt eine PCI-Basisplatine 33 mit einer erfindungsgemäßen Zusammenstellung einzelner Komponenten bzw. Bausteine als Schaltbild.

Die zweite Hardware-Komponente 13 zur Datenverarbeitung in Echtzeit, umfasst einen PC (s. Fig. 1, rechts unten). Der PC ist um eine universelle PCI-Basisplatine 33 erweitert worden. Die Basisplatine 33 weist unter anderem einen field programmable Gate Array 33a (FPGA: Virtex XCV300) und einen digitalen Signalprozessor 35 (DSP D.Modul: Texas Instruments TMS320c6713) auf. Die PCI-Basisplatine 33 mit ihren Komponenten stellt die Echtzeitdatenerfassungs- und Verarbeitungseinheit 13b in der zweiten Hardware-Komponente 13 dar.

Die Verarbeitung der durch das Messsystem 11 gemessenen Daten in Echtzeit ist für die Erzeugung von Rückkopplungssignalen für den Probanden notwendig.

Die Datenerfassungs- und Verarbeitungseinheit 13b (s. Fig. 1) nutzt einen PCI- oder compact-PCI- (cPCI) 34 Erweiterungsbus des PCs und erweitert diesen. Die erfindungsgemäß gezeigte Ausführung gemäß Fig. 3 basiert auf einer PCI-Basisplatine 33. Die Platine 33 stellt über den PCI-Kontroller 33b die Verbindung zwischen dem PCI- oder cPCI-Bus 34 und der Echtzeit-Prozessoreinheit 35, 35a her.

Die Prozessoreinheit 35, 35a umfasst ein Modul mit einem Digitalen Signalprozessor (DSP) 35 mit einem genügend großen Arbeitsspeicher 35a, z. B. mit mindestens 16 MByte. Der digitale Signalprozessor DSP 35 kann über seinen lokalen Datenbus 35b (32 Bit Daten / Adressen) mit dem auf der PCI-Basisplatine 33 vorhandenen Baustein mit programmierbarer Hardware, dem FPGA 33a, Daten austauschen.

Die Ein- und Ausgabekomponenten sind auf einer weiteren Aufsteckplatine, dem Ein- und Ausgabemodul 36, untergebracht. Das Ein- und Ausgabemodul 36 weist eine Lichtwellenleiterschnittstelle 37 zum Lichtwellenleiterkoppler 14, 24 (Fig. 1 und Fig. 2) des Magnetoencephalographen auf sowie analoge Ausgänge 38 für die Stimulation des Probanden und digitale Ein- und Ausgänge für die Ein- und Ausgabe von Zeitmarken, sogenannten Triggern 39.

Das field programmable Gate Array 33a bestimmt durch seine Hardwarekonfiguration das Ein- und Ausgabeverhalten der gesamten Datenerfassungs- und Verarbeitungseinheit 13b und wird über eine JTAG-Schnittstelle 33c konfiguriert.

Das field programmable Gate Array (FPGA) 33a stellt intern eine Liste von Registern zur Verfügung, welche durch Ansprechen einer bestimmten Adresse beschrieben bzw. gelesen werden können.

Der 32 bit Fließkomma DSP TMS320c6713 wird mit 225 MHz getaktet und kann eine theoretische Rechenleistung von 1800 MIPS (Millionen Instruktionen) bzw. 1350 MFLOPS (Millionen Floating Point Operations) je Sekunde leisten.

Am PC der zweiten Hardware-Komponente 13 des MEG-online Systems wird der Leiter 14b in einen Lichtwellenleiterstecker 37 (SC/PC Duplex-Stecker) der Echtzeitdatenerfassungseinheit 13b eingesteckt (s. Fig. 1, rechts unten). Damit sind alle Verbindungen zum MEG-Messsystem 11 hergestellt.

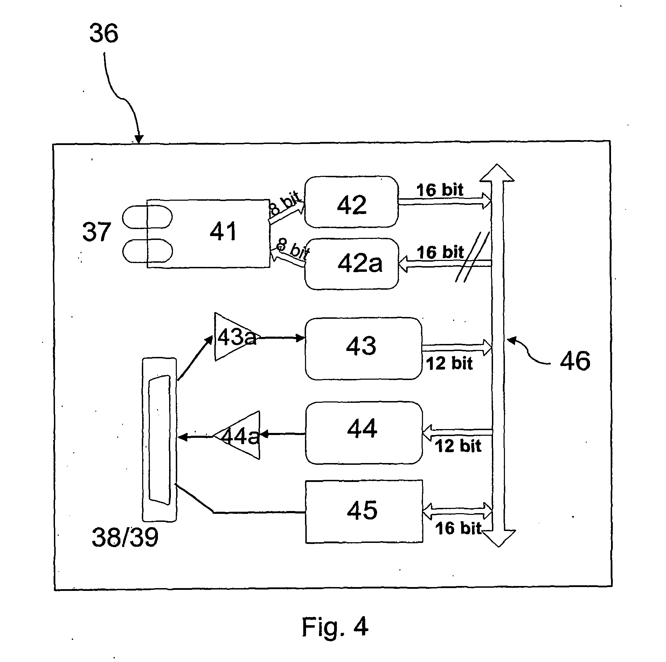

Das Ein- und Ausgabemodul 36 für die Datenerfassungs-und Verarbeitungseinheit 13b ist in Fig. 4 dargestellt.

Das Ein- und Ausgabemodul 36 weist die Schnittstellen zum MEG-Messsystem 11 auf. Die vom MEG-Messsystem 11 als Lichtimpulse kommenden Daten werden über den Lichtwellenleiter 14b an den Stecker 37 des Moduls 36 geleitet. Das Modul weist einen Fiber Optic Transceiver 41 auf. Dieser wandelt die optischen Signale in elektrische Signale um. Der serielle Datenstrom wird mittels eines Hotlink Receiver 42 auf Fehler hin überprüft und zu einem parallelen Datenbyte geformt. Die umgewandelten Daten werden über den Datenbus 46 an das field programmable Gate Array (FPGA) 33a (s. Fig. 3) zur weiteren Verarbeitung übergeben. Ein vorhandener Hotlink Transmitter 42a bleibt für die Datenausgabe ungenutzt. Dies ist durch einen unterbrochenen Datenstrom von Bus 46 zum Hotlink Transmitter 42a angedeutet.

Weiterhin umfasst das Ein- und Ausgabemodul 36 analoge Eingänge, wie den Stecker 39, über den einkommende Daten über einen Verstärker 43a zu einem analog-digitalWandler 43 (4 Kanal, 12 bit ADC, +/- 10 Volt), geführt werden.

Nach der Digitalisierung werden die Daten über den Datenbus 46 vom FPGA 33 eingelesen (s. Fig. 3). Die zur Stimulation auszugebenden Signale werden als elektrische Daten über den Bus 46 in den Digital-AnalogWandler 44 (4 Kanal, 12 bit DAC, +/- 10 Volt) eingeschrieben. Die erzeugten analogen Signale werden nach einer Verstärkung im Verstärker 44a auf den Stecker 38 zwecks Stimulation ausgegeben (s. Fig. 3). Die Daten werden über den Stecker 38 zu einer dritten Hardware-Komponente 15 mit optischer Brille geführt.

Die digitalen Ein- und Ausgänge 45 (16 bit Digital, In / Output) sind an den Stecker 39 geführt und werden für das Ausgeben bzw. das Einlesen von Zeitmarken (Trigger) genutzt.

Die vom MEG-Messsystem 11 als Lichtimpulse einkommenden Daten werden somit vom Photodetektor im Lichtwellenleiterbaustein 37 (Fig. 3) empfangen und in digitale Signale umgewandelt, welche zum FPGA 33a geführt werden. Die Daten werden vom FPGA 33a mittels einstellbarer Filter vorverarbeitet und nach entsprechenden Korrekturen an den digitalen Signalprozessor 35 weitergegeben. Im digitalen Signalprozessor 35 werden die Daten in Echtzeit mit geeigneten und auf den Anwendungszweck für den Probanden angepassten Algorithmen bearbeitet. Auf Grund der Berechnungen werden Daten für ein Ausgangssignal zur Stimulation bereitgestellt. Diese Daten werden wieder in das field programmable Gate Array (FPGA) 33a eingeschrieben, und gelangen über den Bus 46 in den analogen Ausgabebaustein 44 auf dem Ein- und Ausgabeinodul 36. Das Signal wird erneut im Verstärker 44a verstärkt und über den Stecker 38 als Stimulationssignal ausgegeben.

Die gemessenen sowie auch die berechneten Daten werden weiterhin über den PCI Kontroller 33b und den PCI-Bus 34 in den PC (nicht dargestellt) übertragen. Die im PC der zweiten Hardware-Komponente implementierte Software stellt die Daten bzw. Signale online dar, speichert diese ab und kann weitere Auswertungen durchführen. Es ist in diesem Zusammenhang zu betonen, dass die Workstation 11 als erste Hardware-Komponente vorliegend nur noch die Funktion der Steuerung des Messsystems zur Erhebung der Daten vornimmt.

Für weitere Messaufgaben, wie z.B. die Aufzeichnung einiger MEG-Kanäle, stehen vier analoge Eingänge mit 12 bit Auflösung zur Verfügung. Weiterhin stehen vier analoge Ausgänge sowie 16 digitale Ein- und Ausgangskanäle zur Verfügung. Die analogen Ausgänge werden zur Erzeugung der analogen Rückkopplungssignale für.die Stimulations-Experimente genutzt. Es ist dadurch möglich, mit dem Verfahren eine Stimulation eines Probanden, basierend auf in Echtzeit gemessenen und verarbeiteten Hirnaktivitäten, durchzuführen. So können durch das erfindungsgemäße Verfahren die verarbeiteten Daten linear oder nichtlinear transformiert und zeitlich verzögert an den Probanden zurückgeführt werden. Die nichtlineare Transformation kann dabei Daten verschiedener Zeiten miteinander verknüpfen.

Beim Feedback werden dem Probanden Daten zwecks Stimulation zugeführt, welche von den aktuell gemessenen Daten berechnet wurden. Dafür wird die Echtzeitfähigkeit der Datenerfassungs- und Verarbeitungseinheit 13b genutzt, indem zu jedem Abtastzeitpunkt ein neuer Stimulationswert berechnet und auf einen Kanal der analogen Schnittstelle ausgegeben wird.

Das Rückkopplungs- bzw. Stimülationssignal wird dabei entweder von einem MEG-Sensor oder vom Stromdichteverlauf eines Bereiches im Gehirn abgeleitet. Für diese Experimente muss daher der Stromdichteverlauf eines Gehirnareals für jeden Abtastpunkt in Echtzeit berechnet werden. Anschließend wird das Signal bandbreitenbegrenzt und das Rückkopplungssignal berechnet. Diese Berechnungen finden im digitalen Signalprozessor (DSP) 35 statt.

Ein zweites Ausführungsbeispiel behandelt eine Vorrichtung wie in Ausführungsbeispiel 1, allerdings ohne die erste Hardware-Komponente, der Workstation 12.

An Stelle der Workstation 12 speichert die Echtzeitdatenerfassungs- und Verarbeitungseinheit 13b auch die Daten des Messsystems 11. Die Einheit 13b verarbeitet die Daten zudem in Echtzeit. Die Echtzeitdatenerfassungs- und Verarbeitungseinheit 13b speichert die erhobenen und verarbeiteten Daten und steuert darüber hinaus den Messablauf des Messsystems 11 zur Erhebung der Daten. Es ist dann in der Vorrichtung nur eine Verbindungsleitung 11a vorgesehen, über die die Echtzeitdatenerfassungs- und Verarbeitungseinheit 13b vom Messsystem zur Erhebung der Daten 11 Signale erhält. Eine Trennung der Daten ist nicht mehr nötig, da die Aufgaben Speicherung und Verarbeitung der Daten sowie Steuerung des Messsystems von der Echtzeitdatenerfassungs-und Verarbeitungseinheit 13b allein übernommen werden. Über eine weitere Leitung wird das Messsystem durch die Datenerfassungs- und Verarbeitungseinheit 13b gesteuert (nicht dargestellt).

Es ist selbstverständlich möglich, die dargestellte zweite Hardware-Komponente grundsätzlich auch für Berechnungen und Rückführungen von Daten eines EEG-online-Systems zu benutzen.

## Patentansprüche

1. Vorrichtung zur Messung biomedizinischer Daten eines Probanden, umfassend
- ein Messsystem (11) zur Erhebung der Daten eines Probanden,
- eine erste Hardware-Komponente (12) zur Aufzeichnung der Daten;
- mindestens eine Verbindungsleitung (11a, 14c) zur Übertragung der Daten vom Messsystem (11) zur ersten Hardware-Komponente (12), wobei in der Verbindungsleitung (11 a, 14c) zwischen dem Messsystem (11) und der ersten Hardware-Komponente (12) ein Mittel (14, 24) zur Auftrennung der Daten angeordnet ist;
- eine zweite Hardware-Komponente (13,13b), welche die aufgetrennten Daten zugeführt erhält und eine Verarbeitung dieser Daten in Echtzeit ausführt;
- eine dritte Hardware-Komponente, welche die in Echtzeit verarbeiteten Daten von der zweiten Hardware-Komponente (13) erhält und an den Probanden weiterleitet,
**dadurch gekennzeichnet,**
**dass** das Mittel in der Verbindungsleitung (11a, 14c) ein Mittel zur galvanischen Auftrennung ist, welches die durch das Messsystem (11) erhobenen Daten störungsfrei auftrennt und kopiert und,
**dass** die dritte Hardware-Komponente (15) eine Stimulationsbrille und/oder eine Tonquelle umfasst, an welche die von der zweiten Hardware-Komponente (12) erhaltenen Daten zur Stimulation des Probanden mittels Stimulationsbrille und/oder Tonquelle weitergeleitet werden.

2. Vorrichtung nach Anspruch 1,
**gekennzeichnet durch**
ein passives Mittel (14, 24) zur Auftrennung der Daten.

3. Vorrichtung nach einem der vorhergehenden Ansprüche,
**gekennzeichnet durch**
einen Lichtwellenleiterkoppler (14, 24) oder einen galvanischen Trennverstärker als passives Mittel zur Auftrennung der Daten.

4. Vorrichtung nach einem der vorhergehenden Ansprüche, bei der
durch das Mittel zur Trennung der Daten identische Datensätze erzeugt werden.

5. Vorrichtung nach einem der vorhergehenden Ansprüche,
**dadurch gekennzeichnet, dass**
die zweite Hardware-Komponente (13) eine Echtzeitdatenerfassungs- und Verarbeitungseinheit (13b) umfasst.

6. Vorrichtung nach vorhergehendem Anspruch 5,
**dadurch gekennzeichnet, dass**
die Komponenten der Echtzeitdatenerfassungs- und Verarbeitungseinheit (13b) auf einer PCI-Basisplatine (33) angeordnet sind.

7. Vorrichtung nach einem der vorhergehenden Ansprüche 5 oder 6,
**dadurch gekennzeichnet, dass**
die Echtzeitdatenerfassungs- und Verarbeitungseinheit (13b) der zweiten Hardware-Komponente (13) einen field programmable Gate Array (FPGA: 33a) umfasst.

8. Vorrichtung nach einem der vorhergehenden Ansprüche 5 bis 7,
**dadurch gekennzeichnet, dass**
die Echtzeitdatenerfassungs- und Verarbeitungseinheit (13b) der zweiten Hardware-Komponente (13) einen digitalen Signalprozessor (DSP: 35) umfasst.

9. Elektroencephalograph oder Magnetoencephalograph als Vorrichtung nach einem der Ansprüche 1 bis 8.

10. Verfahren zur Echtzeit-Stimulation eines Probanden mit von ihm erhobenen Daten, mit den Schritten:
a) biomedizinische Daten des Probanden werden durch ein Messsystem (11) erhoben,
b) die erhobenen Daten werden durch ein Mittel zur galvanischen Trennung störungsfrei galvanisch vom Messsystem (11) getrennt und kopiert,
c) eine Kopie der Daten wird einer Echtzeitdatenverarbeitungseinheit zugeführt und in Echtzeit verarbeitet,
d) die verarbeiteten Daten des Probanden werden in Echtzeit zum Zweck der Stimulation an den Probanden weitergeleitet, wobei zur Stimulation eine Stimulationsbrille und/oder eine Tonquelle eingesetzt werden.

11. Verfahren nach vorhergehendem Anspruch 10,
**gekennzeichnet durch**
Wiederholung der Schritte a) bis d).

12. Verfahren nach Anspruch 10 oder 11,
bei dem jeweils eine Kopie der erhobenen und der verarbeiteten Daten kontinuierlich gespeichert wird.

13. Verfahren nach einem der vorhergehenden Ansprüche 10 bis 12,
**dadurch gekennzeichnet, dass**
die Daten linear oder nichtlinear transformiert und zeitlich verzögert an den Probanden zurückgeführt werden.

14. Verfahren nach einem der vorhergehenden Ansprüche 10 bis 13,
bei dem eine Vorrichtung nach einem der vorhergehenden Ansprüche 1 bis 9 verwendet wird.

## Claims

1. Apparatus for measuring a subject's biomedical data, comprising
- a measuring system (11) for acquiring subject data,
- a first hardware component (12) for recording the data,
- at least one connection line (11a, 14c) for transmitting the data from the measuring system (11) to the first hardware component (12), a means (14, 24) for splitting the data being arranged in the connection line (11a, 14c) between the measuring system (11) and the first hardware component (12),
- a second hardware component (13, 13b) to which the split data are fed and which processes those data in real time, [and]
- a third hardware component which receives the data processed in real time from the second hardware component (13) and retransmits them to the subject,
**characterized in that**
the means in the connection line (11a, 14c) is a decoupling means which, without interference, splits and copies the data acquired by the measuring system (11), and
**in that** the third hardware component (15) comprises stimulation glasses and/or a sound source to which the data received by the second hardware component (12) are retransmitted to stimulate the subject by means of said stimulation glasses and/or sound source.

2. Apparatus according to Claim 1,
**characterized by**
a passive means (14, 24) for splitting the data.

3. Apparatus according to either of the preceding claims,
**characterized by**
a fibre optic coupler (14, 24) or multicoupler as passive means for splitting the data.

4. Apparatus according to any one of the preceding claims, in which
identical data records are generated by the means for splitting the data.

5. Apparatus according to any one of the preceding claims,
**characterized in that**
the second hardware component (13) comprises a real-time data acquisition and processing unit (13b).

6. Apparatus according to the preceding Claim 5,
**characterized in that**
the components of the real-time data acquisition and processing unit (13b) are arranged on a PCI base board.

7. Apparatus according to either of the preceding Claims 5 and 6,
**characterized in that**
the real-time data acquisition and processing unit (13b) of the second hardware component (13) comprises a field programmable gate array (FPGA: 33a).

8. Apparatus according to any one of the preceding Claims 5 to 7,
**characterized in that**
the real-time data acquisition and processing unit (13b) of the second hardware component (13) comprises a digital signal processor (DSP: 35).

9. Electroencephalograph or magnetoencephalograph as apparatus according to any one of Claims 1 to 8.

10. Method for real-time stimulation of a subject with data acquired from that subject, with the following steps:
a) biomedical data on the subject are acquired by a measuring system (11),
b) the data acquired are split from the measuring system (11) and copied, without interference, by a decoupling means,
c) a copy of the data is fed to a real-time data processing unit and processed in real time,
d) the processed data on the subject are retransmitted to the subject in real time for the purpose of stimulation, stimulation glasses and/or a sound source being used for the stimulation.

11. Method according to the preceding Claim 10,
**characterized by**
repetition of steps a) to d).

12. Method according to Claim 10 or Claim 11,
wherein copies of both acquired and processed data are continuously stored.

13. Method according to any one of the preceding Claims 10 to 12,
**characterized in that**
the data are linearly or non-linearly transformed and fed back to the subject with a time lag.

14. Method according to any one of the preceding Claims 10 to 13,
for which an apparatus according to any one of the preceding Claims 1 to 9 is used.

## Revendications

1. Dispositif de mesure de données biomédicales provenant d'un sujet à examiner, comprenant :
- un système de mesure (11) pour acquérir les données d'un sujet,
- un premier composant de matériau (12) pour enregistrer les donnés ;
- au moins une ligne de raccordement (11a 14c) pour la transmission des données du système de mesure (11) au premier composant de matériau (12), un moyen (14, 24) de séparation des données étant disposé dans la ligne de raccordement (11a, 14c) entre le système de mesure (11) et la premier composant de matériau (12) ;
- un deuxième composant de matériau (13, 13b) qui reçoit les données séparées acheminées et exécute un traitement de ces données en temps réel,
- un troisième composant de matériau qui reçoit les données traitées en temps réel par le deuxième composant de matériau (13) et les retransmet au sujet,
**caractérisé en ce que**
le moyen dans la ligne de raccordement (11a 14c) est un moyen de séparation galvanique qui sépare et copie sans perturbation les données acquises par le système de mesure (11),
**en ce que** le troisième composant de matériau (15) comprend une lunette de stimulation et/ou une source sonore sur laquelle les données obtenues du deuxième composant de matériau (12) sont retransmises pour stimulation du sujet au moyen de la lunette de stimulation et/ou de la source sonore.

2. Dispositif selon la revendication 1
**caractérisé par**
un moyen passif (14, 24) de séparation des données.

3. Dispositif selon l'une des revendications précédentes, **caractérisé par**
un coupleur-transmetteur d'ondes lumineuses (14, 24) ou un amplificateur-séparateur comme moyen passif de séparation des données.

4. Dispositif selon l'une des revendications précédentes, dans lequel
le moyen de séparation des données permet de générer des groupes de données identiques.

5. Dispositif selon l'une des revendications précédentes, **caractérisé en ce que**
le deuxième composant de matériau (13) comprend une unité d'acquisition et de traitement des données en temps réel (13b).

6. Dispositif selon la revendication 5 précédente,
**caractérisé en ce que**
les composants de l'unité de saisie et de traitement des données en temps réel (13b) sont disposés sur une platine de base de PCI (33).

7. Dispositif selon l'une des revendications 5 ou 6 précédentes,
**caractérisé en ce que**
l'unité d'acquisition et de traitement des données en temps réel (13b) du deuxième composant de matériau (13) comprend un réseau logique programmable (field programmable gate array, FPGA, 33a).

8. Dispositif selon l'une des revendications précédentes 5 à 7,
**caractérisé en ce que**
l'unité de saisie et d'acquisition des données en temps réel (13b) du deuxième composant de matériau (13) comprend un processeur de signal numérique (DSP : 35).

9. Electroencéphalographe ou magnétoencéphalographe constituant un dispositif selon l'une des revendications 1 à 8.

10. Procédé de stimulation en temps réel d'un sujet avec des données acquises provenant de celui-ci, comportant les étapes :
a) des données biomédicales du sujet sont acquises par un système de mesure (11),
b) les données acquises sont séparées et copiées par un moyen de séparation galvanique sans perturbation sur le plan galvanique du système de mesure (11),
c) une copie des données est acheminée à une unité de traitement des données en temps réel et est traitée en temps réel,
d) les données traitées du sujet sont retransmises en temps réel au sujet à des fins de stimulation, une lunette de stimulation et/ou une source sonore étant utilisée pour la stimulation.

11. Procédé selon la revendication 10 précédente,
**caractérisé par**
la répétition des étapes a) à d).

12. Procédé selon la revendication 10 ou 11,
dans lequel respectivement une copie des données acquises et des données traitées est enregistrée en continu.

13. Procédé selon l'une des revendications précédentes 10 à 12,
**caractérisé en ce que**
les données sont transformées de façon linéaire ou non linéaire et retransmises au sujet avec un décalage temporel.

14. Procédé selon l'une des revendications 10 à 13 précédentes,
dans lequel on utilise un dispositif selon l'une des revendications 1 à 9 précédentes.
